Europäisches Patentamt

European Patent Office

Office européen des brevets

(11) Publication number: **0 233 847**
**A2**

## (12) EUROPEAN PATENT APPLICATION

(21) Application number: **87830050.8**

(22) Date of filing: **12.02.87**

(51) Int. Cl.⁴: **A 61 C 19/00**

(30) Priority: **21.02.86 IT 333986**

(43) Date of publication of application:
**26.08.87 Bulletin 87/35**

(84) Designated Contracting States:
**CH ES FR GR LI SE**

(71) Applicant: **CASTELLINI S.p.A.**
**Via Saliceto, 22**
**I-40013 Castelmaggiore (Bologna) (IT)**

(72) Inventor: **Castellini, Franco**
**Via Bellinzona, 60**
**I-40135 Bologna (IT)**

(74) Representative: **Lanzoni, Luciano**
**c/o BUGNION S.p.A. Via Farini, 37**
**I-40124 Bologna (IT)**

(54) **Method and apparatus for disinfecting and sterilizing the spray circuits of power driven instruments used in desintry.**

(57) The method and apparatus according to the invention permit of sterilizing power driven instruments used in dentistry, by supplying a given quantity of fluid disinfectant to the spray circuit of the instrument selected, then introducing a rinsing liquid which will follow once the disinfectant has been allowed to remain within the circuit for a length of time dependent upon the operation -i.e. disinfection only or full sterilization.

FIG 1

## Description

METHOD and APPARATUS for DISINFECTING and STERILIZING the SPRAY CIRCUITS of POWER DRIVEN INSTRUMENTS USED in DENTISTRY.

The invention relates to a method and apparatus for disinfecting and sterilizing the spray circuits of power driven instruments used in dentistry.

It is common knowledge that instruments of the kind must be sterilized in order to ensure against the spread of dangerous infections, in particular those transmitted through the bloodstream, such as serum hepatitis.

This problem is especially acute in dentistry, as the mouth is one of the major routes by which such infections are transmitted; in the dental surgery, in fact, instruments are brought into contact daily with the mouths of numerous patients.

The prior art embraces a considerable number of devices or apparatus for disinfecting the detachable parts of instruments (handles, probes &c.) that are brought into direct contact with the teeth, or at all events with infectious media. No such device or apparatus is suitable, however, for the sterilizing of power driven instruments and permanent systems, such as high speed drills and the circuits through which fluids are supplied to their tips, notably, the spray circuit.

The single attachments fitted to the drill will in fact be disinfected at the end of each session of treatment, but no facility exists for disinfection of the spray circuit, which may well trap infectious matter that could be transmitted to the next patient on whom the instrument is used.

Accordingly, the object of the invention is that of remedying this lack of suitable apparatus by means of which to disinfect the spray circuits of power driven instruments used in dental surgery.

The method and apparatus according to the invention, as characterized by the appended claims, envisage the use of a first auxiliary circuit by means of which to supply a fluid disinfectant to the spray circuit of the power driven instrument, and a second auxiliary circuit by means of which the same spray circuit is supplied with a liquid serving to rinse away the fluid disinfectant.

One of the advantages provided by the invention is that the apparatus is both effective and functional. Another advantage of the invention consists in safe operation of the apparatus, inasmuch as the power driven instrument undergoing disinfection remains inhibited from operation until such time as the rinsing stage is completed.

A further advantage of the invention lies in the facility of obtaining either disinfection or full sterilization of the spray circuit, operations which depend simply upon the length of time the fluid disinfectant is allowed to remain in the circuit. Not least among the advantages afforded by the invention is the facility of implementing the entire disinfecting or sterilizing operation automatically. The invention will now be described in detail, by way of example, with the aid of the accompanying drawings, in which:

fig 1 is a block diagram of apparatus according to the invention;

fig 2 is the diagram of a possible embodiment of the electrical circuit in apparatus according to the invention.

With reference to the drawings, 23 denotes the spray circuit of a power driven instrument 2 of the type used in dentistry. Dental surgery equipment will include a given number of such instruments, each one of which connecting with a relative solenoid valve 1 the energization of which is produced by operation of a control switch 19 that shifts when the grip of the instrument 2 is lifted and distanced from its holder in the console.

The solenoid valve 1 controls a line 17a-23 through which water is supplied to the instrument.

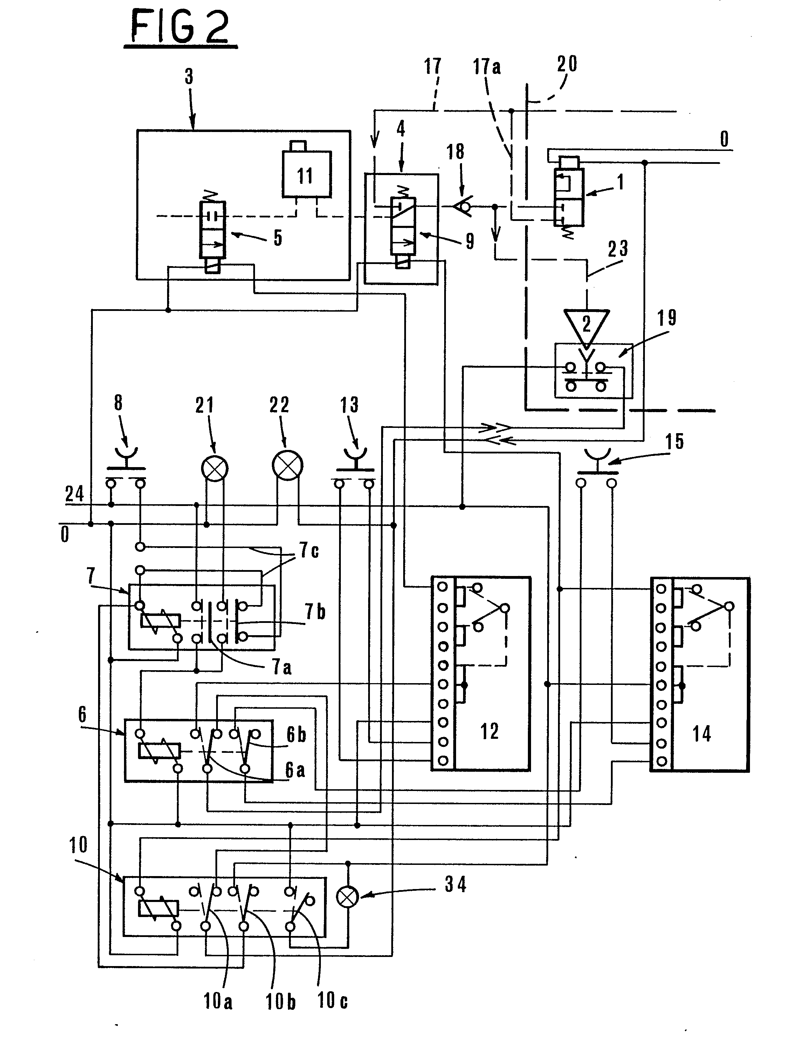

The circuit operated by the switch 19 also comprises a warning light 22, and is wired in with a selector circuit to which each of the single instruments 2 is connected. In fig 2, a broken line 20 separates this conventional section of the wiring from the area to which the disclosure specifically relates.

In a method according to the invention, disinfection or sterilization of the spray circuit of a single power driven instrument involves the steps of:
-breaking the electrical circuit of the instrument 2 between the control switch 19 and solenoid valve 1;
-activation of a fluid circuit 3 from which a given preset quantity of disinfectant is supplied to the spray circuit 23 of the instrument 2;
-activation of a fluid circuit 4 from which rinsing liquid is supplied to the spray circuit 23 of the instrument 2;
-remaking the electrical circuit of the instrument 2 between the control switch 19 and solenoid valve 1, to enable subsequent operation of the instrument.

It is of fundamental importance that the circuit incorporating the control switch 19 remain broken until the rinsing stage has been completed, in order to inhibit operation of a disinfected or sterilized, but as yet unrinsed instrument 2.

The two fluid circuits 3 and 4 will be preset to operate for a given length of time in each instance; in practice, the duration will depend on the fluid capacity of the spray circuit 23 and the type of disinfectant employed.

The disinfectant must be allowed to remain in the spray circuit 23 for a given length of time, and to this end, the circuit 4 supplying the rinse liquid will be switched in only after the circuit 3 which supplies the disinfectant has been deactivated. The length of time effectively spent by the fluid disinfectant in the spray circuit 23 is dependent upon the type of operation required, that is to say, disinfection or sterilization. A few seconds will suffice for disinfection purposes, say 10...20, whereas a full sterilization will require several hours, or even overnight exposure of the circuit 23 to the disinfectant.

Apparatus as disclosed can be integrated into any dental surgery equipment as currently marketed, by implementation of a few simple conversions, the most important of which consists in the wiring-up of two series-connected solenoid operated switches 6 and 10 to the branch of the existing circuit which connects the control switch 19 and the solenoid valve 1.

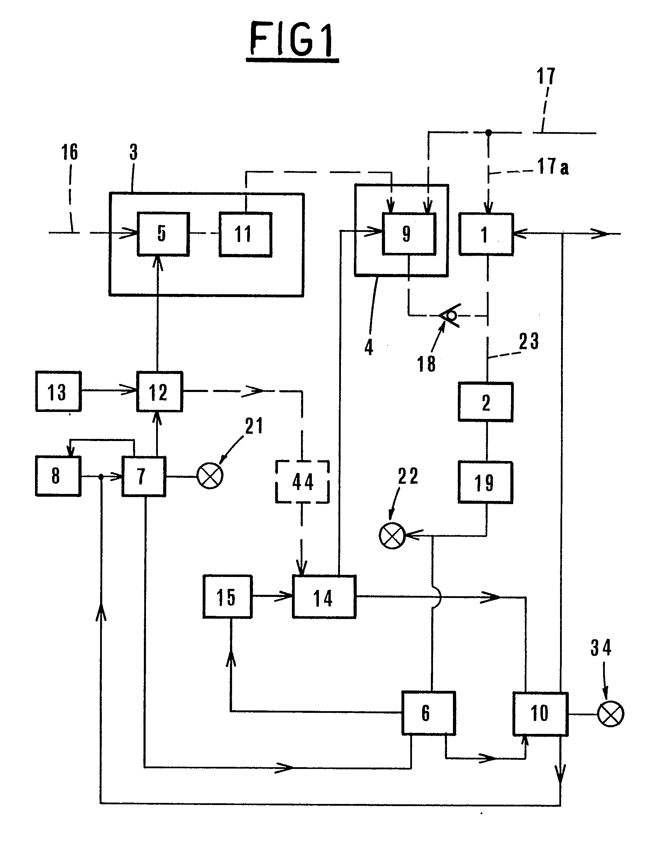

With reference to fig 1, the disinfectant circuit 3 in apparatus according to the invention comprises a first solenoid valve 5 installed on a pipeline 16 carrying pressurized gas, for example compressed air supplied by the circuit of the surgery equipment, and connecting with a tank 11 that contains the fluid disinfectant.

The first solenoid valve 5 is energized by a first timer 12, operated by a relative control, say, a button 13.

7 denotes a solenoid operated bistable switch wired to the first timer 12 and serving to enable it for operation; the switch 7 in question is energized initially by an overall control which likewise takes the form of a button 8 in the drawings. It will be seen in fig 1 that the button 8 is in receipt of an inhibiting signal from this same bistable switch 7, the purpose of which will become apparent.

The switch denoted 7 cascades into the first of the two series-connected solenoid operated switches 6 and 10 mentioned above.

The rinse liquid circuit, denoted 4, comprises a second solenoid valve 9 connecting with a supply of water which, in the example illustrated, is provided by the self-same pipeline 17 as connects with the solenoid valve 1 of the standard surgery equipment. This second solenoid valve 9 also connects with the outlet of the disinfectant tank 11, and via a check valve denoted 18, with the spray circuit 23 of the instrument 2; the check valve 18 serves simply to prevent flow of water from the one solenoid valve 1 to the other 9. Thus, the second solenoid valve 9 acts as a three-way valve by means of which the spray circuit 23 can be supplied either with fluid from the tank 11 or with water from the pipeline 17. Like the first solenoid valve, the second solenoid valve 9 is energized by a timer 14, operated by a relative button 15. Button 15 and timer 14 are both set in the enabled condition by the first solenoid operated switch 6, acting directly and via the second solenoid operated switch 10, respectively. The timer 14 also energizes the solenoid of the switch denoted 10 which, when excited, returns the bistable switch 7 to its at-rest position.

The two timers 12 and 14 will be programmed for the length of time their respective solenoid valves 5 and 9 are to remain open, and taking into account the capacity of the spray circuit 23.

21, 22 and 34 denote warning lights, the first wired to the bistable switch 7 and indicating disinfection or sterilization in progress, the second wired into the circuit downstream of the control switch 19 and indicating the stand-by state of the instrument 2, and the third wired to the second solenoid operated switch 10 and indicating rinse in progress.

As fig 2 illustrates, the overall control button 8 is wired to make and break the energizing circuit of the bistable switch 7.

As to the bistable switch 7 itself, one contact 7a serves to make and break the energizing circuit of the first solenoid operated switch 6, whereas the remaining contact 7b makes the bistable switch's own energizing circuit, denoted 7c, when in one stable position, and makes the circuit to the warning light 21 when moved to the other.

With the bistable switch 7 in the at-rest position (bold line, fig 2), its own energizing circuit 7c is made by the relative contact 7b, whilst the position of the other contact 7a dictates a break in the energizing circuit of the first solenoid operated switch 6.

The first solenoid operated switch 6 has two sets of contacts the first of which, denoted 6a, makes the energizing circuit to the instrument solenoid valve 1 whenever the switch 6 itself is disexcited (bold line, fig 2), and sets the timer 12 in the enabled condition when excited (broken line); the second set of contacts 6b enables the second button 15 when the switch 6 is in excited state.

The first button 13 is wired so as to operate the first timer 12, which in turn is wired into the energizing circuit of the first solenoid valve 5; in effect, this valve 5 acts as a simple on/off switch the at-rest, de-energized position of which (shown in fig 2) shuts off the supply of gas through the incoming pipeline 16.

The second solenoid operated switch 10 has three sets of contacts, the first 10a of which makes the energizing circuit to the instrument solenoid valve 1 whenever the switch 10 itself is disexcited (bold line in fig 2); the second set 10b makes a second energizing circuit to the bistable switch 7 whenever the switch 10 is excited (broken line), and the third set 10c occasions illumination of the relative warning light 34, likewise with the switch 10 in the excited state.

The second button 15 is wired so as to operate the second timer 14, which in turn is wired into the energizing circuit of the second solenoid valve 9, as well as to the second set of contacts 10b at the second solenoid operated switch. The second solenoid valve 9 has two positions: at-rest, in which the disinfectant tank 11 is connected with the inlet of the check valve 18 (see fig 2), and energized, in which the inlet of the check valve 18 is connected with the supply of rinse water entering through pipeline 17.

Apparatus thus embodied can be utilized for each one of the power driven instruments 2 with which the surgery is equipped. The single instrument having been removed from its holder, disinfectant and rinse water will be pumped into its spray circuit using the same power source as that used to operate the instrument itself, accordingly, no description of this aspect is given.

0 and 24, in fig 2, denote electrical power supply to the apparatus.

Operation of the apparatus will now be described, with reference to fig 2 in particular.

In normal operation, a given instrument 2 of the set is lifted and distanced from its control switch 19, which shifts (broken line in fig 2) and makes the energizing circuit of the solenoid valve 1 relative to that instrument. The circuit is also made at the two solenoid operated switches 6 and 10, which for the moment remain disexcited, and the warning light 22 comes on. At this juncture, the dentist can set the instrument 2 in motion by operating its control medium, generally a foot pedal (not illustrated). With the session of treatment over, the need duly arises for disinfection of the spray circuit 23 of the instrument, or instruments 2 utilized. The first step is that of placing the instrument/s requiring disinfection in a container, the reason for which will become apparent; accordingly, the relative solenoid valve/s 1 will be energized, and one has illumination of the warning light 22.

The overall control button 8 is now depressed to activate the apparatus with which the invention is directly concerned, whereupon the solenoid operated bistable switch 7 excites, shifting to the position shown in broken line, in fig 2, and in so doing, breaks that branch 7c of its own energizing circuit to which the button 8 is wired (the bistable switch now remains unaffected by further operation of the button 8, accidentally or otherwise); the first solenoid operated switch 6 is now excited, and one has illumination of the warning light 21 indicating the operational state of the apparatus.

The contacts of the first solenoid operated switch 6 shift following energization (broken line in fig 2), breaking the energizing circuit to the instrument solenoid valve, or valves 1 (thereby isolating the instrument/s 2 altogether, as in fig 1) and enabling the first timer 12 and the second button 15 for subsequent operation.

Depressing the first button 13 causes operation of the first timer 12, the result of which is that the first solenoid valve 5 is energized for a period of time Tx; this will have been programmed accordingly. Compressed air is now admitted through the relative line 16 for the duration of Tx, so as to charge the tank 11 and produce a flow of disinfectant toward the de-energized second solenoid valve 9, which admits the fluid and directs it via the check valve 18 into the spray circuit 23 of the instrument or instruments occupying the container. Once through the check valve 18, disinfectant is prevented from finding its way into the water supply line 17, as the instrument solenoid valve/s 1 are closed.

The fluid disinfectant will now be allowed to remain in the spray circuit, or circuits 23, for a duration commensurate with the type of operation required. Should the operation take place between sessions of treatment, i.e. carried out on different patients, disinfection will generally be sufficient, and the disinfectant need be allowed to remain in the spray circuit/s 23 for no longer than 10...20sec.

In the event of the operation taking place at the end of a day's surgery, full sterilization is to be preferred. In this instance, the best policy will be to place all the instruments 2 in the container, and allow the disinfectant to remain in all the relative spray circuits 23 for some hours. For convenience's sake, the period programmed might simply be that during which the surgery equipment remains idle, daytime or overnight.

With apparatus embodied according to the invention, it becomes possible to switch off the electrical power supply during sterilization, since the circuit configuration that obtains at switch-off will be reassumed automatically when power is restored.

The position of the control switch 19 and the bistable switch 7 will not be affected by isolation from the power supply, and the bistable switch 7 ensures immediate resumption of the excited state of the first solenoid operated switch 6 as power returns. At this stage, only one logical option remains open, namely, that of rinsing out the spray circuit/s 23 with water (in effect, the disinfectant circuit 3 could also be reactivated, though this would serve no practical purpose), given that operation of the instruments 2 is inhibited by the closed position of the instrument solenoid valve 1 and by the break in the circuit 7c to which the overall control button 8 is wired.

Accordingly, depressing the second button 15 will switch in the second timer 14, which operates for a given preset duration Ty, making the energizing circuits to the second solenoid valve 9 and the second solenoid operated switch 10. In its energized state, the solenoid valve 9 connects the inlet of the check valve 18, hence the spray circuits 23, with the water supply line 17. The excited state of the second solenoid operated switch 10 (broken line, fig 2) ensures that the energizing circuits to the instrument solenoid valve/s 1 and the bistable switch 7 remain broken and made, via contacts 10a and 10b respectively, and brings on the warning light 34 to indicate that the rinse is in progress. Further excitation of the bistable switch 7 causes a return to its at-rest position (bold line, fig 2); this disexcites the first solenoid operated switch 6 and extinguishes the warning light 21 on the one hand, and remakes the energizing circuit denoted 7c on the other, although the position of the control button 8 clearly prevents immediate energization. Now disexcited, the first solenoid operated switch 6 isolates the first timer 12 and the second button 15 and remakes the energizing circuit to the instrument solenoid valve/s 1, though this remains broken by the position of the switch contacts denoted 10a. With the rinse time Ty lapsed, the relative timer 14 disexcites the second solenoid operated switch 10. The warning light 34 now extinguishes, a break is produced in the circuit to the bistable switch 7, which remains in the at-rest position, and the energizing circuit to the instrument solenoid valve or valves 1 is restored completely.

Only at this point will the instrument stand-by warning light 22 reappear.

A useful variation in embodiment of the apparatus thus described would be gained by removing the two timer buttons 13 and 15 (or button 15 only) and wiring in a third timer 44 between the two existing timers 12 and 14, programmed for the duration of disinfection/sterilization proper, -i.e. the time the disinfectant is allowed to remain in the spray circuit, or circuits 23. The first timer 12 having been enabled by way of the overall control button 8, this third timer 44 would be triggered by the first timer following the lapse of period Tx, and would in its turn trigger the third timer 14 following the programmed lapse. Thus, operation of the system can be rendered fully automatic.

## Claims

1) Method for disinfecting and sterilizing the spray circuits of power driven instruments used in dentistry, characterized in that it comprises the steps of:
-breaking the conventional circuit by which power is supplied to the control medium (1) of an instrument (2) to be disinfected or sterilized;
-activation of a first auxiliary circuit (3) from which a given preset quantity of disinfectant is supplied to the spray circuit (23) of the power driven instrument (2);
-activation of a second auxiliary circuit (4) from which a given preset quantity of rinsing liquid is supplied to the spray circuit (23) of the power driven instrument (2);
-remaking the conventional circuit by which power is supplied to the control medium (1) of a disinfected or sterilized instrument (2), to enable subsequent operation thereof.

2) Method as in claim 1, wherein the circuit by which power is supplied to the control medium (1) of the disinfected or sterilized instrument (2) is remade only on completion of the rinsing step.

3) Method as in claim 1, wherein the first and second circuits (3, 4) supplying disinfectant and rinse liquid are operated for a given length of time (Tx, Ty) in each instance, the duration of which is programmed according to the capacity of the spray circuit (23) of the power driven instrument (2).

4) Method as in claim 3, wherein the second auxiliary circuit (4) is activated following deactivation of the first auxiliary circuit (3) which supplies the fluid disinfectant, thus allowing the disinfectant to remain in the spray circuit (23) of the power driven instrument (2) for a given duration.

5) Method as in claim 1, wherein the length of time spent by the fluid disinfectant in the spray circuit (23) of the power driven instrument (2) is dependent upon the type of operation, namely disinfection or full sterilization, to which the instrument must be subjected.

6) Method as in claim 1, wherein the steps of breaking the conventional circuit by which power is supplied to the control medium (1) of the instrument (2) and activating the first auxiliary circuit (3) to supply fluid disinfectant are implemented simultaneously.

7) Apparatus for disinfecting and sterilizing the spray circuits of power driven instruments used in dentistry, characterized in that it can be readily integrated into standard dental surgery equipment, and in that it comprises:
-a first auxiliary circuit (3), from which fluid disinfectant is supplied to the spray circuit (23) of an instrument (2) to be disinfected/sterilized, served by a first solenoid valve (5) interlocked to a first solenoid operated switch (6) the excitation of which, produced by the operation of an overall control medium (8), breaks the circuit by which power is supplied to the control medium (1) of the instrument (2);
-a second auxiliary circuit (4), from which rinsing liquid is supplied to the spray circuit (23) of the instrument (2), which is served by a second solenoid valve (9) interlocked to a second solenoid operated switch (10).

8) Apparatus as in claim 7, wherein energization of the first and second solenoid valves (5, 9) is triggered by the first and second solenoid operated switches (6, 10) in conjunction with programmable first and second timers (12, 14) operated by relative control media (13, 15), and excitation of the first solenoid switch (6) enables the control medium (15) of the second timer (14), which in turn is wired into the energizing circuit of the second solenoid operated switch (10).

9) Apparatus as in claim 7, wherein excitation of the first solenoid operated switch (6) is produced by the overall control medium (8) in conjunction with a solenoid operated bistable switch (7) capable of breaking that branch of its own energizing circuit into which the overall control medium (8) is wired, and wherein the second solenoid operated switch (10) is designed, when excited, to produce a further break in the circuit by which power is supplied to the control medium (1) of the instrument (2), and when disexcited, to occasion excitation of the bistable switch (7) by-passing the overall control medium (8).

10) Apparatus as in claim 7, wherein energization of the first solenoid valve (5) is triggered by the first solenoid operated switch (6) in conjunction with a programmable timer (12) operated by a respective control medium (13), and energization of the second solenoid valve (9) is triggered by the second solenoid switch (10) in conjunction with a second programmable timer (14) wired into the energizing circuit of the second solenoid operated switch (10) and operated by the first timer (12), on completion of its programmed time lapse, through the agency of a third timer that is programmed according to the length of time the fluid disinfectant is required to remain in the spray circuit (23) of the power driven instrument (2).

11) Apparatus as in claim 7, wherein the first solenoid valve (5) provides on/off control for a supply of pressurized gas entering the apparatus through a pipeline (16) and directed into a tank (11) that contains the fluid disinfectant and is connected with the spray circuit (23) of the power driven instrument (2) by way of the second solenoid valve (9) which, connected in turn with a supply of rinse liquid entering through a further pipeline (17), and with the inlet of a check valve (18) affording access to the spray circuit (23), functions as a logic element by way of which the spray circuit can be supplied either with disinfectant or with rinse liquid.

12) Apparatus as in claim 7, applicable to standard dental surgery equipment having a plurality of power driven instruments (2) connected up to a circuit enabling their selection, wherein the circuitry of the apparatus controlling supply, first, of fluid disinfectant, and thereafter, of rinsing liquid to the spray circuit (23) of a given instrument (2), is interlocked to the circuit enabling selection of the instruments.

# FIG1

FIG 2

0233847